# EUROPEAN PATENT APPLICATION

(11) **EP 4 703 753 A1**
(43) Date of publication of application: **04.03.2026**
(21) Application number: 24275099.0
(22) Date of filing: 28.08.2024
(51) Int. Cl.: G01R 33/38, G01R 33/3815

(54) **CRYOGENIC SYSTEM AND MAGNETIC RESONANCE DEVICE**

(71) Applicant: Siemens Healthcare Limited, Camberley, Surrey GU15 3YL (GB)
(72) Inventor: ALEY, Nicholas, Maldon Essex, CM96NF (GB); TAYLOR, Owen, Appleton, OX13 5LQ (GB)
(74) Representative: Siemens Healthineers Patent Attorneys

(57) **Abstract**

The invention relates to a cryogenic system (30) for a magnetic resonance device (10), comprising a magnet arrangement (11) including at least one superconducting magnet (12), a first cryocooler (31a) thermally connected to the at least one superconducting magnet (12), a switching device (38), and a second cryocooler (31b) configured to cool a component of the magnet arrangement (11) in dependence of the switching device (38), wherein the switching device (38) is configured to enable cooling of the component of the magnet arrangement (11) via the second cryocooler (31b) when a temperature of the component of the magnet arrangement (11) exceeds a predefined temperature level, and to disable cooling of the component of the magnet arrangement (11) via the second cryocooler (31b) when the temperature of the component of the magnet arrangement (11) is below the predefined temperature level. The invention further relates to a magnetic resonance device (10), comprising an inventive cryogenic system (30).

## Description

Independent of the grammatical term usage, individuals with male, female or other gender identities are included within the term.

The latest generation of magnetic resonance devices strives to avoid large helium reservoirs in which superconducting magnet coils are immersed. Some magnetic resonance devices, also referred to as "dry" systems, operate without a liquid cryogen bath for the superconducting magnet coils. These "dry" systems typically comprise a single cryocooler which is thermally connected to the superconducting magnet coils via a solid thermal conductor, e. g. a high purity metal.

In comparison to "dry" systems, the "cold mass" of conventional magnet resonance devices (i. e. "wet" systems) is much easier to cool from room temperature to about 4K, because the helium reservoir can be filled with liquid helium. During operation of a magnetic resonance device, the superconducting magnet needs to be maintained at a temperature level below the superconducting temperature of the superconducting magnet coils. Superconducting magnet coils comprising low temperature superconducting materials typically exhibit superconducting temperatures below 5 K. At these low temperatures, a cooling capacity of the cryocooler will typically be limited to about one Watt.

Typical cryocoolers known from magnetic resonance devices can maintain the superconducting magnet coils at a temperature level of about 4 K during operation of the magnetic resonance device. However, the limited cooling capacity at these low temperatures results in a high time expenditure for cooling the superconducting magnet coils from room temperature to operating temperature. For example, a conventional two-stage cryocooler may require up to two weeks to cool a 1.5 T magnetic resonance device down to operating temperatures. In case of a 7.0 T magnetic resonance device, the same two-stage cryocooler may require up to 120 days. These long cooling periods are often problematic for customers who are waiting for the delivery and installation of a new magnetic resonance device. Furthermore, if a superconducting magnet quenches, it may heat up to over 50 K and needs to be cooled down again. This process can take several days, which is undesirable for the operator of the magnetic resonance device.

In the past, pre-cooling loops have been used that can be filled or flushed with liquid nitrogen or other fluids to reduce the time required for cooling at the production sites. These pre-cooling loops can be set up relatively easily at the production sites due to the existing liquid cryogen infrastructure. However, using pre-cooling loops at the customer site is typically not practical.

It is an object of the invention to reduce a time required for cooling of a superconducting magnet of a magnetic resonance device.

This object is achieved by a cryogenic system and a magnetic resonance device according to the invention. Further advantageous embodiments are specified in the dependent claims.

The inventive cryogenic system is configured for use in a magnetic resonance device. The cryogenic system comprises a magnet arrangement including at least one superconducting magnet, a first cryocooler thermally connected to the at least one superconducting magnet, a switching device, and a second cryocooler configured to cool a component of the magnet arrangement in dependence of the switching device.

The magnet arrangement may comprise a main magnet of the magnetic resonance device. Particularly, the magnet arrangement may comprise one or more superconducting magnets or one or more superconducting magnet coils of the magnetic resonance device. In a preferred embodiment, the magnet arrangement comprises at least two superconducting magnet coils. The at least two superconducting magnet coils may be rotationally symmetric or comprise rotationally symmetric bodies. It is conceivable that the at least two superconducting magnet coils comprise a cylindrical or solenoidal shape. The terms superconducting magnet and superconducting magnet coil are used interchangeably herein.

The one or more superconducting magnet coils of the magnet arrangement may define a common axis. Preferably, the common axis defined by one or more superconducting magnet coils corresponds to a cylinder axis and/or an axis of rotational symmetry of the main magnet and/or the magnet arrangement.

According to an embodiment, the one or more superconducting magnet coils are mechanically coupled and/or mechanically connected. Preferably, the one or more superconducting magnet coils are mechanically connected in such a way to form a cohesive or coherent structure.

For example, the one or more superconducting magnet coils may be integrally bonded. Preferably, at least two superconducting magnet coils are integrally bonded to at least one spacer arranged between the at least two superconducting magnet coils. According to an embodiment, the at least two superconducting magnet coils are bond to opposing sides or faces of the at least one spacer via an adhesive, particularly an epoxy resin.

The magnet arrangement may comprise a magnet support structure configured to provide mechanical support to the at least one superconducting magnet. Particularly, the magnet support structure may be configured to maintain a predefined spatial arrangement of at least two superconducting magnet coils. According to an embodiment, the magnet support structure is configured to be mechanically connected to a support structure, particularly an outer vacuum chamber, of a magnetic resonance device comprising the inventive cryogenic system.

The at least one superconducting magnet may represent an inner magnet of the magnet arrangement.

The magnet arrangement may comprise one or more other components, such as a thermal buffer, a thermal radiation shield, a magnet support structure, a shield tube, an outer magnet (i. e. one or more shield coils) or the like.

It is conceivable that the magnet arrangement is circumferentially enclosed in an outer vacuum chamber of a magnetic resonance device comprising the inventive cryogenic system. The outer vacuum chamber may represent a vessel which is substantially impermeable to fluids, such as a liquid or a gaseous cryogen. The outer vacuum chamber may enclose the cryogenic system and the components of the magnet arrangement, such as the thermal radiation shield, the shield tube, the at least one superconducting magnet, and the like. The vacuum chamber may represent a double-walled hollow cylinder comprising an outer shell and an inner shell. The outer shell and the inner shell of the vacuum chamber may be connected by annular end pieces. The magnet arrangement may be enclosed between the outer shell and the inner shell of the outer vacuum chamber. The inner shell of the outer vacuum chamber may correspond to a patient bore of a magnetic resonance device comprising the inventive cryogenic system.

In a preferred embodiment, the magnet arrangement is configured to provide a magnetic field suitable for use in a magnetic resonance imaging examination. Particularly, the magnet arrangement may be configured to provide a static or main magnetic field (i. e. a SO-field) of the magnetic resonance device. It is conceivable that a maximum magnetic field strength of the magnetic field is between 0.1 and 9 Tesla, for example about 0.5 Tesla, 1.5 Tesla, 3 Tesla, or 7 Tesla.

The at least one superconducting magnet may comprise or consist of a superconducting material. For example, the at least one superconducting magnet may comprise or consist of a low temperature superconducting material, such as Nb₃Sn, Nb₃Al, and NbTi. However, it is also conceivable that the at least one superconducting magnet comprises or consists of a high temperature superconducting material. Examples of high temperature superconducting materials are rare earth barium copper oxide (REBCO) materials or a combination of such materials, such as may be provided by a number of different yttrium barium copper oxide (YBCO) compounds. High temperature superconducting materials typically exhibit higher critical or superconducting temperatures in comparison to low temperature superconducting materials. Thus, an efficiency of the cryocooler (and associated cooling equipment) may favourably be increased and operational costs of the inventive cryogenic system may be reduced when using superconducting magnets comprising high temperature superconducting materials in comparison to low temperature superconducting materials.

The first cryocooler may be configured to maintain the at least one superconducting magnet at a temperature close to or lower than a superconducting temperature of a superconducting material of the at least one superconducting magnet. For example, the superconducting temperature of the at least one superconducting magnet may be in the range of 3 K to 100 K, preferably in the range of 3 K to 6 K, 5 K to 10 K, 30 K to 60 K, 60 K to 90 K, or 90 K to 120 K. The first cryocooler, but also the second cryocooler, may be implemented as a pulse tube refrigerator, a Gifford-McMahon refrigerator, a Stirling cryocooler, a Joule-Thomson cryocooler, or the like.

The first cryocooler may be thermally and mechanically connected to the at least one superconducting magnet via a thermal bus. For example, the thermal bus may comprise or consist of a solid thermal conductor configured to thermally and mechanically connect the first cryocooler to the at least one superconducting magnet. Particularly, the thermal bus may be configured to thermally and mechanically connect a cooling stage of the cryocooler to the at least one superconducting magnet.

The first cryocooler may comprise a cold head with one or more cooling stages. In case the cold head comprises multiple cooling stages, each cooling stage may have a different temperature level. For example, the cold head of the first cryocooler may comprise at least a first stage and a second stage. The second stage may be thermally and mechanically connected to the at least one superconducting magnet. The first stage may be thermally and mechanically connected to a component of the magnet arrangement different from the at least one superconducting magnet. Preferably, a temperature level of the first stage exceeds a temperature level of the second stage. For example, the first stage may be configured to provide a temperature in the range between 40 K to 180 K, preferably in the range between 40 K to 60 K, 60 K to 100 K, or 100 K to 180 K. The second stage may be configured to provide a temperature level close to the superconducting temperature of the at least one superconducting magnet.

It is conceivable that the at least one superconducting magnet is thermally and mechanically connected to the first cryocooler via a thermal bus. For example, the thermal bus may be configured to thermally and mechanically connect the at least one superconducting magnet to a heat exchanger of a cold head of the first cryocooler, particularly a heat exchanger of a second stage of the cold head of the first cryocooler.

According to an embodiment, the thermal bus is mechanically connected to the first cryocooler and/or the at least one superconducting magnet via a form-locking connection, a force-locking connection and/or a material bond. For example, the thermal bus may be screwed, clamped, bolted and/or glued to the first cryocooler and/or the at least one superconducting magnet. Preferably, the mechanical connection between the thermal bus and the cryocooler, but also the mechanical connection between the thermal bus and the at least one superconducting magnet, is configured to allow for thermal energy to be transported from the at least one superconducting magnet to the cryocooler.

According to the invention, the switching device is configured to enable cooling of the component of the magnet arrangement via the second cryocooler when a temperature of the component of the magnet arrangement exceeds a predefined temperature level, and to disable cooling of the component of the magnet arrangement via the second cryocooler when the temperature of the component of the magnet arrangement is below the predefined temperature level.

The switching device may be configured to control a transport of thermal energy from the component of the magnet arrangement to the second cryocooler. For example, the switching device may be configured to activate and deactivate the second cryocooler. However, it is also conceivable that the switching device is configured to control a transport of thermal energy from the component of the magnet arrangement to the second cryocooler. Particularly, the switching device may comprise a heat switch configured to selectively enable or disable a transfer of heat energy between two objects, such as the second cryocooler and the component of the magnet arrangement.

A heat switch may be configured to conduct heat energy between two surfaces, for example a surface of the second cryocooler and a surface of the component of the magnet arrangement. The heat switch may be configured to control the temperature of the component of the magnet arrangement, for example, by enabling it to be cooled via the second cryocooler when the temperature of the component of the magnet arrangement exceeds the predefined temperature level.

The heat switch may be configured as a passive heat switch or an active heat switch (i. e. an actively controlled heat switch). Some preferred examples of possible technologies for heat switches are gas-gap heat switches, superconducting heat switches, or mechanical heat switches. However, heat switch may also be based on other technologies. For example, the heat switch may be configured as a magneto-strictive heat switch, or a heat switch based on magneto-thermal conductivity.

Gas-gap heat switches may comprise a gas-filled chamber configured to transfer heat energy between two surfaces, for example a surface of the second cryocooler and a surface of the component of the magnet arrangement. The transfer of heat energy may be adjusted by changing the pressure of the gas, which affects its thermal conductivity.

Superconducting heat switches may comprise a superconducting material configured to conduct heat energy between the two surfaces. The heat transfer may be adjusted by applying a magnetic field to the superconductor, which causes it to lose its superconductivity and resist heat flow.

Mechanical heat switches may comprise a movable contact or thermal bridge configured to physically connect or disconnect the two surfaces. The heat transfer may be adjusted by moving the contact in or out of contact with at least one of the two surfaces. It is also conceivable that the mechanical heat switch and/or the component of the magnet arrangement exhibit a temperature-dependent expansion, causing a section of the of the mechanical heat switch and/or the component of the magnet arrangement to expand and make contact with the second cryocooler, when the temperature of the component of the magnet arrangement exceeds the predefined temperature level.

The heat switch may be configured to allow for thermal energy to be exchanged between the second cryocooler and the component of the magnet arrangement. Particularly, the heat switch may be configured to allow for thermal energy to be exchanged between the second cryocooler and the component of the magnet arrangement in dependence of a temperature dependent property of the component of the magnet arrangement. Furthermore, the heat switch may be configured to impede or prevent an exchange of heat energy between the second cryocooler and the component of the magnet arrangement in dependence of a temperature dependent property of the component of the magnet arrangement. The temperature dependent property may comprise a thermal expansion, a pressure, a pliability, an electrical conductivity, or the like.

According to an embodiment, the switching device is configured as temperature-dependent switch. Particularly, the switching device may be configured to activate or deactivate the second cryocooler in dependence of the temperature of the component of the magnet arrangement.

In some embodiments, the switching device is configured to intermittently provide a thermal and mechanical connection between the second cryocooler and one or more components of the magnet arrangement in dependence of a temperature level of the one or more components of the magnet arrangement.

In other embodiments, the switching device is configured to activate or deactivate the second cryocooler in dependence of the temperature level of the one or more components of the magnet arrangement. In these embodiments, the second cryocooler may be permanently attached or mechanically connected to the one or more components of the magnet arrangement.

In a preferred embodiment, the predefined temperature level differs from an operating temperature of the component of the magnet arrangement.

An inventive cryogenic system may favourably allow for the second cryocooler to be selectively operated depending on a predefined boundary condition. For example, the operation of the second cryocooler may be restricted to pre-cooling processes, cooling after a quench or ramp down of the at least one superconducting magnet and/or when the predefined temperature level of the component of the magnet arrangement is exceeded. Thus, a parallel operation of the first cryocooler and the second cryocooler may favourably be restricted to times when high cooling capacities are required, which may improve an overall efficiency for cooling and/or reduce operational costs of the cryogenic system.

Furthermore, the switching device may be configured to reduce or prevent a power consumption of the second cryocooler by disabling it or thermally disconnecting it from the component of the magnet arrangement when no cooling is needed. Thus, the second cryocooler may favourably support cooling of the at least one superconducting magnet and other components of the magnet arrangement when high cooling capacities are required, and remain out of operation during normal operation of the magnetic resonance device comprising the inventive cryogenic system.

According to an embodiment of the inventive cryogenic system, the second cryocooler is mechanically and thermally connected to the component of the magnet arrangement. The switching device is configured to activate the second cryocooler when the temperature of the component of the magnet arrangement exceeds the predefined temperature level, and to deactivate the second cryocooler when the temperature of the component of the magnet arrangement is below the predefined temperature level.

The second cryocooler may be permanently attached to the component of the magnet arrangement. For example, a heat exchanger of a cooling stage of the second cryocooler may be mechanically attached to one or more components of the magnet arrangement via a form-locking connection, a force-locking connection and/or a material bond. It is also conceivable that the second cryocooler or the heat exchanger of the cooling stage of the second cryocooler is mechanically connected to a solid thermal conductor or a thermal bus. The solid thermal conductor or thermal bus may be mechanically connected to the one or more components of the magnet arrangement.

A thermal bus or a solid thermal conductor may comprise or consist of a material with high thermal conductivity, such as a metal (e. g. aluminium, copper, silver, gold, or the like), particularly a high-purity metal. The thermal bus or solid thermal conductor may comprise any suitable shape. Preferably, the thermal bus or solid thermal conductor comprises one or more metal parts in the shape of a braid, a rod, a plate, a mesh, a rail, or a combination thereof, forming a continuous thermal conductor.

As described above, the switching device may be configured to activate or deactivate the second cryocooler in dependence of a temperature of the component of the magnet arrangement.

For example, the switching device may comprise a sensor configured to acquire an information indicative of the temperature of the component of the magnet arrangement and a control unit configured to activate and deactivate the second cryocooler in dependence of the information indicative of the temperature of the component of the magnet arrangement. The sensor and the control unit may be configured in accordance with an embodiment described below.

In a further example, the switching device may be configured to only activate the second cryocooler during a pre-cooling process, a ramp up of the at least one superconducting magnet, or a high-performance imaging sequence expected to exceed a cooling capacity of the first cryocooler. Thus, the cryogenic system may be configured to activate the second cryocooler in dependence of a status of the magnetic resonance device comprising the cryogenic system. In this case, the predefined temperature level may represent an expected temperature level of the component of the magnet arrangement. Thus, a sensor configured to acquire an information indicative of a temperature of the component of the magnet arrangement may favourably be omitted.

According to an embodiment, the switching device comprises an electrically conductive element configured to detect a thermal expansion of one or more components of the magnet arrangement. The electrically conductive element may be configured to form a closed loop when the temperature of the one or more components exceeds the predefined temperature level. For example, the thermal expansion of a material of the one or more components of the magnet arrangement may cause two sections of the electrically conductive element to make an electrical connection. The closed loop may provide an electrical signal indicative of the temperature of the component of the magnet arrangement to a control unit configured to activate and deactivate the second cryocooler. However, it is also conceivable that the second cryocooler is directly energized via a current provided when the electrically conductive element forms a closed loop.

In permanently attaching the second cryocooler to the one or more components of the magnet arrangement, a good thermal contact between the second cryocooler and the one or more components of the magnet arrangement may favourably be provided. Thus, a heat transport rate between the one or more components of the magnet arrangement and the second cryocooler may favourably be improved in comparison to embodiments relying on an intermittent mechanical and thermal connection between the second cryocooler and the component of the magnet arrangement.

According to an embodiment of the inventive cryogenic system, the switching device comprises a heat switch. The second cryocooler is thermally connected to the component of the magnet arrangement via the heat switch. The heat switch is configured to thermally connect the second cryocooler and the component of the magnet arrangement when the temperature of the component of the magnet arrangement exceeds the predefined temperature level, and to thermally isolate the second cryocooler from the component of the magnet arrangement when the temperature of the component of the magnet arrangement is below the predefined temperature level.

The heat switch may be configured in accordance with an embodiment described above.

The heat switch may be arranged between the second cryocooler and the component of the magnet arrangement. Preferably, the heat switch is mechanically attached to the second cryocooler and/or the component of the magnet arrangement. The heat switch may form a part of the second cryocooler or the component of the magnet arrangement. It is conceivable that the heat switch is configured to temporary or intermittently thermally connect the second cryocooler to the component of the magnet arrangement. According to an embodiment, the heat switch is configured to intermittently mechanically couple the second cryocooler and the component of the magnet arrangement.

The heat switch may be configured to thermally connect the second cryocooler to the component of the magnet arrangement, when the temperature of the component exceeds a predefined value. For example, cooling of the component of the magnet arrangement via the second cryocooler may favourably be prevented if the temperature of the component of the magnet arrangement is below the predefined temperature level. Thus, an operation of the second cryocooler may favourably restricted to times of high cooling demand, e. g. during a pre-cooling process, a ramp up process of the at least one superconducting magnet (i. e. after a quench or an intentional ramp down) and/or during high-performance imaging sequences.

If the second cryocooler is operated at temperatures exceeding the superconducting temperature of the at least one superconducting magnet, a cooling capacity and/or a cooling efficiency of the second cryocooler may exceed a cooling capacity and/or a cooling efficiency of the first cryocooler. Thus, the second cryocooler may favourably reduce a time for cooling, but also operational costs, of a magnetic resonance device comprising the cryogenic system. Furthermore, the second cryocooler may favourably reduce a cool down time required for cooling the at least one superconducting magnet from room temperature to about 4 K by a factor of about 2.5 to 3 compared to a single two-stage cryocooler.

The heat switch may be configured to disable cooling of the component of the magnet arrangement via the second cryocooler. Thus, operating costs for the second cryocooler may be saved in comparison to cryogenic systems comprising a plurality of continuously operating cryocoolers.

A heat switch may provide a robust and self-contained solution for automatically engaging and disengaging the second cryocooler in dependence of a temperature dependent property of the component of the magnet arrangement. Thus, any effort associated with sensors or a control unit may favourably be avoided.

In a preferred embodiment of the inventive cryogenic system, an operating temperature of the component of the magnet arrangement thermally connected to the second cryocooler via the heat switch exceeds a superconducting temperature of the at least one superconducting magnet.

An operating temperature of the component of the magnet arrangement may be characterized by a temperature, particularly a mean temperature or an average temperature, of the component of the magnet arrangement during a regular operation of the magnetic resonance device comprising the inventive cryogenic system.

Different components of the magnet arrangement may be maintained at different temperatures during an operation of the magnetic resonance device. For example, the at least one superconducting magnet and the magnet support structure may be maintained at a temperature corresponding substantially to the superconducting temperature of the at least one superconducting magnet. However, other components of the magnet arrangement, such as the thermal radiation shield, a shield tube, a thermal buffer, but also parts of a support structure of any of these components, may be maintained at temperatures exceeding the superconducting temperature of the at least one superconducting magnet.

For example, the at least one superconducting magnet may be maintained at a temperature of about 4 K, while the thermal radiation shield may be maintained at a temperature in the range of 40 K to 60 K, preferably about 42 K, 43 K, or 45 K.

The second cryocooler may be thermally connected to one or more components of the magnet arrangement, whose operating temperature exceeds the superconducting temperature of the at least one superconducting magnet during operation of the magnetic resonance device. For example, the second cryocooler may be operated during an application of high-performance imaging sequences, when the cooling capacity of the first cryocooler may not be sufficient to maintain desired temperature levels of the components of the magnet arrangement. Thus, the second cryocooler may favourably be operated in addition to the first cryocooler to prevent an undesirable heating of components of the magnet arrangement during high-performance imaging sequences. Particularly, the second cryocooler may favourably be operated in addition to the first cryocooler to prevent heating of the thermal radiation shield due to gradient coil induced heating.

The heat switch may be configured to thermally connect the second cryocooler to the component of the magnet arrangement for pre-cooling purposes or during a ramp up of the at least one superconducting magnet (e. g. following a quench or a ramp down of the at least one superconducting magnet), but also during operation of the magnetic resonance device comprising the inventive cryogenic system.

In operating the second cryocooler at a temperature level above the superconducting temperature of the at least one superconducting magnet, an energy efficiency of the second cryocooler may be increased substantially in comparison to an energy efficiency of the first cryocooler. Thus, costs associated with cooling down a magnetic resonance device comprising the inventive cryogenic system after a quench or ramp down, but also as part of an initial cool down, may favourably be decreased.

According to an embodiment of the inventive cryogenic system, the predefined temperature level is in the range of 40 K to 120 K.

Preferably, the predefined temperature level exceeds the superconducting temperature of the at least one superconducting magnet. Particularly, the predefined temperature level may exceed the superconducting temperature of the at least one superconducting magnet by several degrees Kelvin or several ten degrees Kelvin.

In an example, the at least one superconducting magnet comprises a low temperature superconducting material. In this case, the predefined temperature level may be in the range of 40 K to 50 K, 50 K to 60 K, or 60 K to 70 K. However, the predefined temperature level may also be lower than 40 K or higher than 70 K depending on the operating temperature of the component of the magnet arrangement.

In a further example, the at least one superconducting magnet comprises a high temperature superconducting material. In this case, the predefined temperature level may be in the range of 70 K to 80 K, 80 K to 90 K, 90 K to 100 K, 100 K to 110 K, or 110 K to 120 K. However, the predefined temperature level may also be lower than 70 K or higher than 120 K depending on the operating temperature of the component of the magnet arrangement.

In operating the second cryocooler at temperature levels in excess of the superconducting temperature of the at least one superconducting magnet, an energy efficiency and/or a cooling capacity of the second cryocooler may favourably be increased in comparison to the first cryocooler. Thus, a cool down time for the cryogenic system may favourably be decreased, while saving operational costs of the cryogenic system.

Furthermore, the heat switch may be configured to thermally disconnect the second cryocooler from the component of the magnet arrangement when the temperature of the component of the magnet arrangement drops below the predefined temperature level. Thus, an operation of the second cryocooler may favourably be prevented, when the temperature of the component of the magnet arrangement drops below a minimum temperature provided by the second cryocooler.

According to an embodiment of the inventive cryogenic system, the component of the magnet arrangement thermally connected to the second cryocooler via the heat switch is at least one of a thermal buffer, a thermal radiation shield, a magnet support structure, a shield tube, and/or the at least one superconducting magnet.

The cryogenic system may comprise components of the magnet arrangement. In a preferred embodiment, the magnet arrangement comprises a thermal radiation shield configured to shield the at least one superconducting magnet from stray fields generated by a gradient system of the magnetic resonance device comprising the inventive cryogenic system. Particularly, the thermal radiation shield may be configured to reduce a transport of thermal energy to the at least one superconducting magnet. A transport of thermal energy may be characterized by a heat transport mechanism such as thermal radiation, thermal conduction, but also thermal convection. The thermal radiation shield, particularly an outer wall of the thermal radiation shield, may circumferentially encompass the at least one superconducting magnet.

The thermal radiation shield may form a vessel enclosing the at least one superconducting magnet, but also a shield tube. In a preferred embodiment, the thermal radiation shield comprises an outer wall, an inner wall and annular end walls connecting the outer wall and the inner wall. Particularly, the thermal radiation shield may form a double-walled hollow cylinder enclosing the at least one superconducting magnet, but also the shield tube, between the outer wall, the inner wall and the annular end walls. According to an embodiment, the at least one superconducting magnet is arranged between the outer wall and the inner wall of the thermal radiation shield. For example, the at least one superconducting magnet may be enclosed in a vessel formed by the outer wall, the inner wall as well as the annular end walls of the thermal radiation shield.

The thermal radiation shield may comprise an axis of rotational symmetry oriented in parallel to or corresponding to an axis of rotational symmetry defined by the at least one superconducting magnet.

Preferably, the thermal radiation shield comprises or consists of a material with a high electrical conductivity and/or a high thermal conductivity. For example, the thermal radiation shield may consist of copper or aluminium, particularly high purity aluminium. The thermal radiation shield may also comprise gold, platinum, silver, or other metals with a high electrical conductivity and/or high thermal conductivity. In one embodiment, the thermal radiation shield is coated or galvanized with a material having a high electrical conductivity and/or high thermal conductivity.

The thermal radiation shield may be configured to shield the at least one superconducting magnet from (electro-)magnetic fields, particularly an electromagnetic field generated by a gradient system of a magnetic resonance device. For example, the thermal radiation shield may be configured to allow for eddy currents or screening currents to be generated along an electrically conducting surface of the thermal radiation shield in dependence of the electromagnetic field. Particularly, the thermal radiation shield may comprise an electrically conductive layer allowing for a generation of eddy currents in dependence of a primary electromagnetic field, e. g. a gradient magnetic field, particularly a stray field of a gradient magnetic field. The generated eddy currents may generate a secondary (electro-)magnetic field opposing the primary electromagnetic field. Particularly, the primary electromagnetic field and the secondary electromagnetic field may cancel each other out to some extent, thus providing a shielding effect.

According to an embodiment of the inventive cryogenic system, the component of the magnet arrangement thermally connected to the second cryocooler via the heat switch is a shield tube.

The shield tube may be configured in accordance with an embodiment described in EP23275129.

For example, the magnet arrangement may comprise at least one spacer mechanically connected to the thermal radiation shield and the shield tube, wherein the at least one spacer is configured to maintain a predefined distance between the inner wall of the shield structure and the shield tube. The at least one spacer and/or the shield tube may comprise a rigid material arranged in such a way to improve a transfer of mechanical energy between the thermal radiation shield and the shield tube. It is also conceivable that the at least one spacer is arranged in such a way to improve a transfer of mechanical energy between the shield structure and the shield tube.

In a further example, the thermal radiation shield comprises a plurality of layers, wherein at least one layer of the plurality of layers is configured to modify a mechanical resonance behaviour of the thermal radiation shield in dependence of a mechanical resonance behaviour of the shield tube. Particularly, the at least one layer of the thermal radiation shield may be configured to increase a stiffness of the thermal radiation shield.

In a preferred embodiment, the magnet arrangement comprises a gradient system including at least one gradient coil configured to generate a gradient magnetic field in a volume circumferentially encompassed by the inner wall of the thermal radiation shield. The shield tube is spaced from the gradient coil in such a way to reduce a screening current induced in the shield tube via a stray field of the gradient magnetic field.

The shield tube may comprise or consist of a material having an intermediate electrical conductivity at a temperature of 300 K. According to an embodiment, the shield tube comprises or consists of a material having a thermal conductivity of less than 120 *W*/(*m* · *K*), less than 100 *W*/(*m* · *K*), or less than 80 *W*/(*m* · *K*) at a temperature of 300 K. For example, the shield tube may consist of steel or stainless steel. However, the shield tube may comprise or consist of other materials, particularly metals having a high electrical conductivity, such as copper or aluminium.

The shield tube may be mechanically supported by the thermal radiation shield. Preferably, the shield tube is mechanically supported by the inner wall of the thermal radiation shield.

A shield tube according to an embodiment described above may favourably allow for a reduction of heat loads on cryogenically cooled components of a magnetic resonance device without compromising image quality. Furthermore, a shield tube may comprise or consist of a material having an intermediate electrical conductivity, thus favourably reducing manufacturing costs of the cryogenic system and/or the magnet arrangement. Particularly, a shield tube may reduce or prevent an occurrence of long time-constant eddy currents, which can negatively impact image quality.

In thermally connecting the second cryocooler to the shield tube via the heat switch, heat loads caused by the gradient system (e. g. due to gradient coil induced heating, particularly during high-performance imaging sequences) may favourably be reduced or prevented in a particular efficient manner.

In a preferred embodiment, the shield tube is maintained at a lower temperature in comparison to other components of the magnet arrangement, such as the thermal radiation shield. For example, the second cryocooler may be configured to maintain the shield tube at a lower temperature than the operating temperature of the thermal radiation shield and/or other components of the magnet arrangement. A shield tube maintained at a lower temperature may exhibit a lower electrical resistance and therefore a lower power dissipation. A lower temperature and a higher electrical conductivity of the shield tube may improve a shielding of the at least one superconducting magnet.

Furthermore, a lower power dissipation (e. g. a lower heating) on the shield tube may result in a more stable temperature during scanning which may in turn improve an efficiency of eddy current compensation algorithms.

A thermal buffer may represent a thermal storage. Preferably, the thermal buffer may comprise a high thermal capacity. For example, the thermal buffer may comprise a liquid or gaseous volume of a cryogen. A cryogen may represent a fluid with a low boiling point, such as Argon, Nitrogen, Neon, Helium, or the like. The cryogen may be contained in a sealed container. However, it is also conceivable that the thermal buffer comprises a solid material with a high thermal capacity, a liquid-solid phase change material, and/or a gas-liquid phase change material. The thermal buffer may be thermally connected to one or more components of the magnet arrangement. Preferably, the thermal buffer is configured to maintain a desired temperature level of the one or more components of the magnet arrangement even if a cooling capacity of the first cryocooler and/or the second cryocooler is insufficient. For example, the thermal buffer may be configured to maintain the desired temperature level of the one or more components of the magnet arrangement during high-performance imaging sequences.

The second cryocooler may be configured to cool one or more of the components of the magnet arrangement during an initial cool down of the cryogenic system, but also following a quench or a ramp down of the at least one superconducting magnet.

The heat switch may be configured to thermally disconnect or separate the second cryocooler from one or more of the components of the magnet arrangement mentioned above, once the temperature level of the one or more components of the magnet arrangement drops below the predefined temperature level. Particularly, the heat switch may be configured to thermally disconnect or separate the second cryocooler from components of the magnet arrangement, whose operating temperature is below a minimum temperature level provided by the second cryocooler. Furthermore, the heat switch may be configured to selectively thermally disconnect or separate the second cryocooler from components of the magnet arrangement, whose operating temperature is maintained by the first stage and/or the second stage of the first cryocooler.

An inventive cryogenic system may favourably accelerate a cool down of a magnetic resonance device comprising an inventive cryogenic system without causing increased operational costs associated with running multiple cryocoolers during a regular operation of the magnetic resonance device.

A second cryocooler thermally connected to one or more of the above-mentioned components of the magnet arrangement via the heat switch may favourably allow for cooling or pre-cooling of the one or more components of the magnet arrangement to temperatures different from a temperature level provided by a cooling stage of the first cryocooler. Thus, cooling capacities provided by the first cryocooler and the second cryocooler can be used more efficiently.

According to an embodiment of the inventive cryogenic system, the heat switch is a passive heat switch configured to thermally connect the component of the magnet arrangement to the second cryocooler in dependence of a temperature dependent property of the component of the magnet arrangement.

In a preferred embodiment, the passive heat switch is configured as a gas-gap heat switch or a mechanical heat switch according to an embodiment described above.

It is conceivable that the passive heat switch is configured to provide a thermal and mechanical connection between the second cryocooler and one or more components of the magnet arrangement in dependence of a thermal expansion of a material of the one or more components of the magnet arrangement.

For example, the passive heat switch may comprise an expansion element configured to thermally and mechanically connect the one or more components to the second cryocooler when a temperature of the one or more components and/or the expansion element exceeds the predefined temperature level. Furthermore, the expansion element may be configured to thermally and mechanically disconnect the one or more components of the magnet arrangement from the second cryocooler when a temperature of the one or more components of the magnet arrangement and/or the expansion element drops below the predefined temperature level. The expansion element may be attached to the one or more components of the magnet arrangement and share a temperature with the one or more components of the magnet arrangement. Particularly, the expansion element may be thermally and mechanically connected to the one or more components of the magnet arrangement according to an embodiment describe above.

A passive heat switch may favourably provide a robust and/or inexpensive solution for thermally connecting the second cryocooler to the component of the magnet arrangement in dependence of the predefined temperature level of the component of the magnet arrangement.

According to a further embodiment, the inventive cryogenic system and/or the heat switch comprise a control unit and a sensor. The sensor is configured to determine a temperature dependent property of the component of the magnet arrangement. The heat switch is an actively controlled heat switch, and the control unit is configured to control the heat switch to thermally connect the component of the magnet arrangement to the second cryocooler in dependence of the temperature dependent property of the component of the magnet arrangement.

The sensor may be configured to acquire an information indicative of a temperature of the component of the magnet arrangement and output a signal indicative of a temperature of the component of the magnet arrangement. For example, the sensor may be configured to measure the temperature of the component of the magnet arrangement. However, the sensor may also be configured to determine a temperature dependent property of the component of the magnet arrangement, such as a mechanical stress within a material, a pressure, a thermal expansion, an electrical conductivity, or the like.

The control unit may be configured to control the heat switch to thermally connect the component of the magnet arrangement to the second cryocooler in dependence of the signal provided by the sensor. For example, the control unit may be configured to output a control signal controlling the heat switch to thermally connect the second cryocooler to one or more components of the magnet arrangement. The control unit may comprise a signal connection configured to receive the signal from the sensor. The signal connection may be configured as a wireless signal connection or a wired electrical or optical signal connection.

The heat switch may comprise an actuator element configured to thermally and mechanically connect the second cryocooler to the component of the magnet arrangement in dependence of a control signal provided by the control unit. The actuator element may comprise a drive unit configured to convert the control signal into a mechanical movement. For example, the actuator element may be configured as an electric motor, a bimetal actuator, a hydraulic actuator, a pneumatic actuator, an electrochemical actuator, an electromechanical actuator, a piezo actuator, a magneto-strictive actuator, a shape memory alloy actuator, an electroactive polymer actuator, or the like.

According to an embodiment, the actuator element is configured to thermally and mechanically connect the second cryocooler to one or more components of the magnet arrangement by pressing a solid thermal conductor onto a surface of the second cryocooler and the one or more components of the magnet arrangement. Thus, a thermal bridge may be provided thermally connecting the second cryocooler to the one or more components of the magnet arrangement.

For example, the actuator element may be configured as a hydraulic or pneumatic actuator configured to move the solid thermal conductor or thermal bridge onto the surface of the second cryocooler and the surface of the one or more components of the magnet arrangement. In a further example, the actuator element comprises a piezo actuator or an electric motor configured to mechanically move the solid thermal conductor or thermal bridge onto the surface of the second cryocooler and the surface of the one or more components of the magnet arrangement.

The control unit may represent a standalone component or form a part of a main control unit of a magnetic resonance device comprising the inventive cryogenic system.

An actively controlled heat switch may favourably allow for the second cryocooler to be thermally connected to the one or more components of the magnet arrangement in a particularly accurate and/or reproduceable manner.

According to an embodiment of the inventive cryogenic system, the first cryocooler is a two-stage cryocooler having a first stage and a second stage, wherein a temperature level of the first stage exceeds a temperature level of the second stage.

The first stage of the first cryocooler may be configured to provide a temperature level in the range of 40 K to 60 K. In a preferred embodiment the first stage is configured to provide a temperature of about 43 K. The second stage of the first cryocooler may be configured to provide a temperature level in the range of 4 K to 6 K, particularly about 4.2 K.

The first stage of the first cryocooler may be configured to provide a cooling capacity of about 50 W to 60 W. The second stage of the first cryocooler may be configured to provide a cooling capacity of about 1 W to 2 W. The cooling capacities provided by the first stage and the second stage of the first cryocooler may increase with increasing temperature levels. For example, the first stage of the first cryocooler may be configured to provide up to 80 W of cooling capacity at 80 K and the second stage of the first cryocooler may be configured to provide up to 18 W of cooling capacity at 20 K. The second cryocooler may correspond to the first cryocooler. However, one or more cooling stages of the second cryocooler may be operated at different temperatures compared to the cooling stages of the first cryocooler.

Of course, the temperature levels provided by the first stage and the second stage of the first cryocooler may be raised if the at least one superconducting material comprises a high temperature superconducting material. In this case, the first cryocooler may comprise a single stage configured to provide a temperature level below or close to the superconducting temperature of the at least one superconducting magnet.

In a preferred embodiment of the inventive cryogenic system, the second cryocooler is configured as a single-stage cryocooler comprising a single stage.

The second cryocooler may comprise a single stage or single cooling stage. Preferably, the single stage of the second cryocooler is configured to provide a cooling capacity of about 80 W at a temperature of 80 K. However, the single stage of the second cryocooler may be configured to provide a higher cooling capacity at a higher temperature level. In raising the minimum temperature level provided by the single stage of the cryocooler, a cooling efficiency of the second cryocooler may favourably be increased.

In an alternative embodiment, the single stage of the second cryocooler is configured to provide a cooling capacity of about 18 W at a temperature of 20 K. A second cryocooler configured to provide a temperature level of about 20 K may favourably support cooling or pre-cooling of the at least one superconducting magnet down to a lower temperature in comparison to cryocoolers operating at a higher temperature. Thus, the required time for cooling the at least one superconducting magnet may favourably be reduced at the expense of efficiency and/or operational costs of the cryogenic system.

A single-stage cryocooler may be more robust, but also less expensive, in comparison to a multi-stage cryocooler.

According to an embodiment of the inventive cryogenic system, a temperature level of the single stage of the second cryocooler exceeds a temperature level of the second stage of the first cryocooler.

According to an embodiment, the temperature level of the second stage of the first cryocooler ranges between 4 K and 5 K. However, the temperature level of the second stage of the first cryocooler may be higher depending on the material composition of the at least one superconducting magnet. The temperature level of the single stage of the second cryocooler may exceed the temperature level of the second stage of the first cryocooler by at least 10 K, 20 K, 30 K, 40 K, 50 K, 60 K, 70 K, 80 K, 90 K, 100 K, 110 K, or more.

According to a preferred embodiment, the temperature level of the single stage of the second cryocooler matches a temperature level of the first cooling stage of the first cryocooler or exceeds the temperature level of the first stage of the first cryocooler.

For example, the first stage of the first cryocooler and the single stage of the second cryocooler are configured to provide a temperature level in the range of 40 K to 60 K, particularly about 43 K.

In a further example, the temperature level of the single stage of the second cryocooler exceeds the temperature level of the second stage of the first cryocooler by at least 10 K, 20 K, 30 K, 40 K, 50 K, or more.

Operating two cryocoolers at a temperature of about 4 K to 5 K may provide an increased cooling capacity at lower temperatures. However, the efficiency of a cryocooler diminishes significantly with decreasing temperatures at the coldest stage of the cryocooler. Thus, providing a second cryocooler configured to provide cooling at a temperature exceeding the temperature of the second stage of the first cryocooler may favourably increase a cooling efficiency and/or reduce costs associated with the cool down of a magnetic resonance device comprising the inventive cryogenic system, while still allowing for a reduction of the cool down time.

According to a further embodiment of the inventive cryogenic system, the second cryocooler is a multi-stage cryocooler comprising at least a first stage and a second stage. A temperature level of the first stage of the second cryocooler exceeds a temperature level of the second stage of the second cryocooler, and the temperature level of the second stage of the second cryocooler exceeds a lowest temperature level provided by the first cryocooler.

The lowest temperature level provided by the first cryocooler may correspond to the temperature level of the second stage of the first cryocooler.

It is conceivable that the temperature level of the second stage of the second cryocooler matches or exceeds the temperature level of the first stage of the first cryocooler. However, it is also conceivable that the temperature level of the second stage of the second cryocooler exceeds the temperature level of the second stage of the first cryocooler but falls below the temperature level of the first stage of the first cryocooler. The temperature level of the first stage of the second cryocooler may exceed the temperature level of the first stage of the first cryocooler.

The manufacturing costs of a multi-stage cryocooler may exceed the manufacturing costs of a single-stage cryocooler. However, a multi-stage cryocooler may provide a distribution of cooling capacities that is better adapted to the needs of the one or more components of the magnet arrangement. For example, the first stage of the second cryocooler may operate at higher temperatures and higher cooling capacities, while the second stage of the second cryocooler may still contribute to a cool down of the magnet arrangement even below a temperature of the first stage of the first cryocooler. Thus, an inventive cryogenic system comprising a multi-stage second cryocooler may favourably provide a desired compromise between investment costs and operating costs.

According to a further embodiment of the inventive cryogenic system, a cooling capacity of a cooling stage of the second cryocooler exceeds a cooling capacity of the first stage and/or the second stage of the first cryocooler.

The cooling stage of the second cryocooler may correspond to the single stage, the first stage, or the second stage of the second cryocooler according to an embodiment described above.

For example, the second cryocooler may be configured as a single-stage cryocooler and the cooling stage of the second cryocooler may correspond to a single stage of the second cryocooler. The cooling capacity of the single stage of the second cryocooler may exceed a cooling capacity of the second stage of the first cryocooler, but also a cooling capacity of the first stage of the first cryocooler.

In another example, the second cryocooler is configured as a two-stage cryocooler and the cooling stage of the second cryocooler corresponds to the second stage of the second cryocooler. The cooling capacity of the second stage of the second cryocooler may exceed a cooling capacity of the second stage of the first cryocooler, but also a cooling capacity of the first stage of the first cryocooler. Preferably, the cooling capacity of the first stage of the second cryocooler exceeds the cooling capacity of the first stage and the second stage of the first cryocooler.

A cooling capacity and/or an efficiency of the second cryocooler may increase with an increasing temperature level provided by the cooling stage of the second cryocooler.

A second cryocooler comprising a cooling stage configured to provide a cooling capacity exceeding the cooling capacity of the first stage and/or the second stage of the first cryocooler may favourably provide a good compromise or an economic optimum between a required time for cooling and operating costs associated with the cryogenic system.

The inventive magnetic resonance device is configured to acquire magnetic resonance data from an object positioned in an imaging region of the magnetic resonance device.

Preferably, the magnetic resonance device is configured to acquire magnetic resonance image data, particularly diagnostic magnetic resonance image data, from the object positioned within the imaging region. The object may be an inanimate object or a patient, particularly a human or an animal.

The inventive magnetic resonance device may represent a closed bore scanner. A closed bore scanner may comprise a substantially cylindrical bore circumferentially enclosing the imaging region. The magnet arrangement of the closed bore scanner may comprise a main magnet including one or more solenoidal superconducting magnet coils (or solenoidal superconducting magnets) circumferentially encompassing the imaging region along an axial direction or an axis of rotational symmetry of the cylindrical bore. The one or more superconducting magnet coils may comprise a superconducting wire having a negligible electrical resistance at (or below) a superconducting temperature. A direction of a main magnetic field provided by the one or more superconducting magnet coils may be oriented substantially in parallel to a direction of access of the object to the imaging region and/or the axial direction of the cylindrical bore.

In other embodiments, the inventive magnetic resonance device may be configured as an open bore scanner, a C-type scanner, a single-sided scanner, or the like.

The inventive magnetic resonance device may comprise further components required for a proper operation of the magnetic resonance device. For example, the magnetic resonance device may comprise an outer vacuum chamber, a magnet support structure, one or more thermal radiation shields, a shield tube and/or a magnet arrangement according to an embodiment described above. In certain embodiments, the magnetic resonance device and/or the magnet arrangement comprise a cryogen vessel.

A cryogen vessel may be configured for storing or preserving a fluid, particularly a cryogen, at a desired temperature level. Preferably, the fluid or cryogen exhibits a low boiling point. Examples of suitable fluids or cryogens are Argon, Nitrogen, Neon, Helium, or the like. The desired temperature level may substantially correspond to a superconducting temperature of the at least one superconducting magnet.

The inventive magnetic resonance device may represent a "dry" system comprising a minimum of cryogen or no cryogen at all. For example, the inventive magnetic resonance device comprises one or more small cryogen vessels thermally connected to the main magnet via a solid thermal conductor. The one or more small cryogen vessels may contain a volume of less than 10 l, less than 5 I, or less than 1 l of cryogen. According to an embodiment of the inventive magnetic resonance device, a cryogen vessel is omitted, and the magnet arrangement is cooled entirely via thermal conduction.

The inventive magnetic resonance device comprises a cryogenic system according to an embodiment described above.

The inventive magnetic resonance device shares the advantages of the inventive cryogenic system according to an embodiment described above.

Further advantages and details of the present invention may be recognized from the embodiments described below as well as the drawings. The figures show:
- Fig. 1: a schematic representation of an embodiment of an inventive magnetic resonance device,
- Fig. 2: a schematic representation of an embodiment of an inventive magnetic resonance device comprising an inventive cryogenic system,
- Fig. 3: a schematic representation of an embodiment of an inventive magnetic resonance device comprising an inventive cryogenic system,
- Fig. 4: a schematic representation of an embodiment of an inventive magnetic resonance device comprising an inventive cryogenic system,
- Fig. 5: a schematic representation of an embodiment of an inventive magnetic resonance device comprising an inventive cryogenic system,
- Fig. 6: a schematic representation of an embodiment of an inventive magnetic resonance device comprising an inventive cryogenic system,
- Fig. 7: a schematic representation of an embodiment of an inventive magnetic resonance device comprising an inventive cryogenic system.

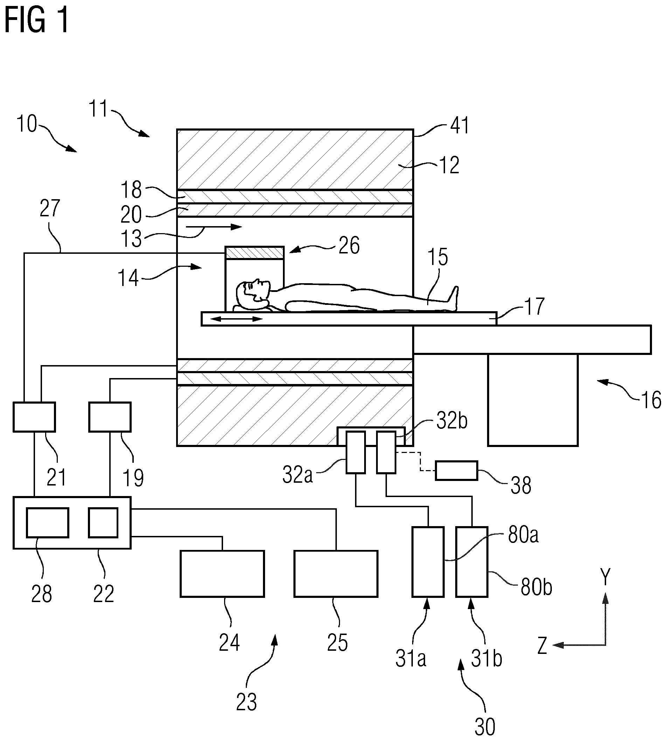

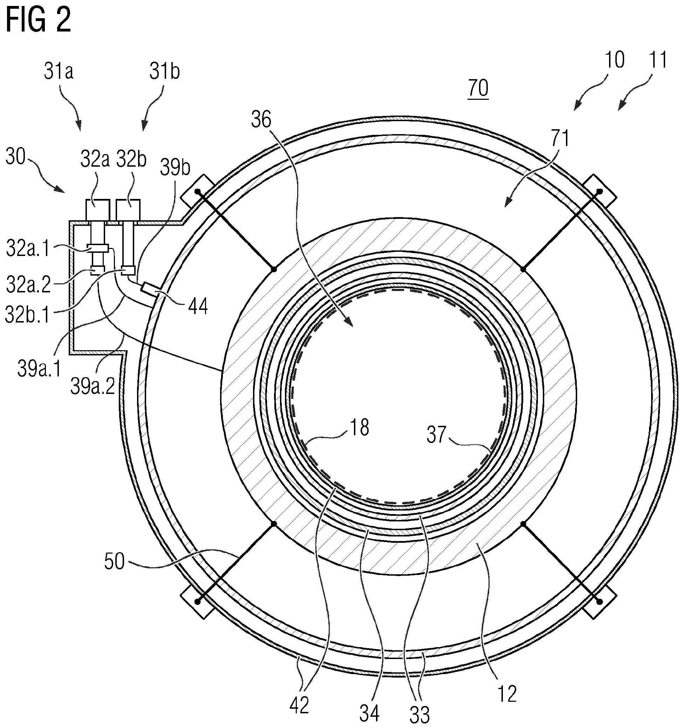

Fig. 1 shows an embodiment of a magnetic resonance device 10 according to the invention. The magnetic resonance device 10 comprises a static field magnet or main magnet 12 including one or more superconducting magnets (not shown) configured to provide a homogenous, static magnetic field 13 (B0 field). The static magnetic field 13 permeates an imaging region 14 configured to receive an imaging object, such as a patient 15. The imaging region 14 may correspond to a patient bore 37 (see Fig. 2) configured to accommodate a patient 15 during a magnetic resonance measurement. The imaging region 14 is encompassed by the main magnet 12 in a circumferential direction. The main magnet 12 may form a part of a magnet arrangement 11.

The magnetic resonance device 10 may comprise a patient positioning device 16 configured to transport the patient 15 into the imaging region 14. Particularly, the patient positioning device 16 may be configured to transport a diagnostically relevant body region of the patient 15 into an imaging volume (not shown) of the magnetic resonance device 10. The magnet arrangement 11 and other components of the magnetic resonance device 10 may be concealed in a housing 41.

The magnetic resonance device 10 may comprise a gradient system including one or more gradient coils 18. The one or more gradient coils 18 may be configured to generate gradient magnetic fields in different, preferably orthogonally oriented, spatial directions. The gradient magnetic fields can be used for spatial encoding of magnetic resonance signals or magnetic resonance data acquired during a magnetic resonance measurement. The one or more gradient coils 18 can be activated or controlled via an appropriate control signal provided by a gradient control unit 19.

The magnetic resonance device 10 may further comprise an integrated radiofrequency antenna 20 (i. e. a body coil). The radiofrequency antenna 20 may be activated or controlled via a radiofrequency control unit 21. The radiofrequency control unit 21 may be configured to control the radiofrequency antenna 20 to generate a high frequency magnetic field and emit radiofrequency excitation pulses into the imaging region 14. The magnetic resonance device 10 may further comprise a local coil 26. The local coil 26 may be positioned on or in proximity to a diagnostically relevant body region of the patient 15. The local coil 26 may be configured to emit radiofrequency excitation pulses into the patient 15 and/or receive magnetic resonance signals from the patient 15. It is conceivable, that the local coil 26 is controlled via the radiofrequency controller 21.

Preferably, the magnetic resonance device 10 comprises a control unit 22 configured to control the magnetic resonance device 10. The control unit 22 may comprise a processing unit 28 configured to process magnetic resonance signals and reconstruct magnetic resonance images. The processing unit 28 may also be configured to process input from a user of the magnetic resonance device 10 and/or provide an output to a user. For this purpose, the processing unit 28 and/or the control unit 22 can be connected to a display unit 24 and an input unit 25 via a suitable signal connection. For a preparation of a magnetic resonance measurement, preparatory information, such as imaging parameters or patient information, can be provided to the user via the display unit 24. The input unit 25 may be configured to receive information and/or imaging parameters from the user. The display unit 24 and the input unit 25 may form a part of a user interface 23 configured to enable the user to interact with the magnetic resonance device 10.

The magnetic resonance device 10 further comprises two cryocoolers 31a and 31b configured to cool the main magnet 12, but also other components of the magnet arrangement 11. The cryocoolers 31a and 31b may each comprise one or more compressors 80a, 80b supplying pressurized gas or cryogen to the cryocoolers 31a and 31b. However, it is also conceivable that the cryocoolers 31a and 31b share a single compressor 80.

In the example depicted in Fig. 1, each cryocooler 31a, 31b includes a cold head 32a, 32b comprising one or more cooling stages (not shown). According to an embodiment, a second stage of the cold head 32a of the first cryocooler 31a is thermally connected to the main magnet 12, while a first stage of the cold head 32a of the first cryocooler 31a is thermally connected to other components of the magnet arrangement 11, such as the thermal radiation shield 33, a shield tube 34, a buffer unit, or the like (see Figs. 2 to 6). The second cryocooler 31b may include a cold head 32b comprising a single cooling stage. The second cryocooler 31b is configured to cool a component of the magnet arrangement 11 in dependence of the switching device 38.

The switching device 38 is configured to enable cooling of a component of the magnet arrangement 11 via the second cryocooler 31b when a temperature of the component of the magnet arrangement 11 exceeds a predefined temperature level, and to disable cooling of the component of the magnet arrangement 11 via the second cryocooler 31b when the temperature of the component of the magnet arrangement 11 is below the predefined temperature level.

Of course, the magnetic resonance device 10 may comprise further components and/or functions which are common in magnetic resonance devices. The general operation of a magnetic resonance device 10 is known to those skilled in the art, so a more detailed description is omitted.

Fig. 2 shows a sectional view of an embodiment of an inventive magnetic resonance device 10. The magnetic resonance device 10 comprises an outer vacuum chamber 42 providing an outer enclosure for the components of the magnet arrangement 11. The outer vacuum chamber 42 separates a surrounding environment 70 from a vacuum region 71 encompassed by the outer vacuum chamber 42. In a preferred embodiment, the outer vacuum chamber 42 forms a double-walled hollow cylinder comprising an outer shell and an inner shell. The components of the magnet arrangement 11 may be housed within or enclosed by the outer shell and the inner shell of the outer vacuum chamber 42. The inner shell of the outer vacuum chamber 42 may form the patient bore 37 encompassing the imaging region 36 in a circumferential direction.

In the illustrated embodiment, the magnet arrangement 11 comprises a thermal radiation shield 33 and a shield tube 34. The thermal radiation shield 33 is configured as a double-walled hollow cylinder, whereas the shield tube 34 is configured as a tube or a hollow cylinder. The shield tube 34 and the main magnet 12 are enclosed between an outer wall and an inner wall of the thermal radiation shield 33. Thus, the main magnet 12 is confined or enclosed by the outer wall of the thermal radiation shield 33 and the shield tube 34.

The magnet arrangement 11 may comprise a support structure configured to mechanically attach the main magnet 12 to the outer vacuum chamber 42. In the depicted example, the magnet arrangement 11 comprises one or more suspension elements 50 or suspension rods mechanically connected to the outer shell of the outer vacuum chamber 42 and the main magnet 12. The suspension elements 50 may extend through passages or holes in the thermal radiation shield 33 to provide a mechanical connection between the outer shell of the outer vacuum chamber 42 and the main magnet 12 or a magnet support structure configured to support the main magnet 12.

In the embodiment depicted in Fig. 2, the magnetic resonance device 10 comprises a first cryocooler 31a including a cold head 32a and second cryocooler 31b including a cold head 32b (only the cold heads 32a and 32b are shown in detail). The cold heads 32a and 32b are mounted on the outer shell of the outer vacuum chamber 42.

The first cryocooler 31a may be configured to cool the main magnet 12, the thermal radiation shield 33, the shield tube 34, but also other components of the magnet arrangement 11, such as a cryogen vessel (not shown).

In the depicted example, the second cryocooler 31b comprises a single stage 31b.1. The single stage 31b. 1 of the second cryocooler 31b is thermally connected to the thermal radiation shield 33 via a heat switch 44. The heat switch 44 is configured to thermally connect the second cryocooler 31b and the thermal radiation shield 33 when a temperature of the thermal radiation shield 33 exceeds a predefined temperature level. The heat switch 44 is also configured to thermally isolate the second cryocooler 31b from the thermal radiation shield 33 when the temperature of the thermal radiation shield 33 is below the predefined temperature level.

In some embodiments, the second cryocooler 31b may be thermally connected to further components of the magnet arrangement 11, such as the shield tube 34, or the main magnet 12, via the heat switch 44.

The cryocoolers 31a and 31b typically comprise one or more compressors 80 supplying pressurized gas or cryogen to the cold heads 32a and 32b (see Fig. 1). According to the embodiment shown in Fig. 2, the first cryocooler 31a includes a cold head 32a comprising a first stage 32a.1 and a second stage 32a.2. Preferably, the first stage 32a.1 of the cold head 32a is thermally connected to the thermal radiation shield 33, whereas the second stage 32a.2 of the cold head 32a is thermally connected to the main magnet 12.

In one example, the first cooling stage 32a.1 of the first cryocooler 31a is configured to provide a temperature level of about 43 K, whereas the second cooling stage 32a.2 of the first cryocooler 31a is configured to provide a temperature level of about 4.2 K. The first stage 32a.1 and the second stage 32a.2 of the first cryocooler 31a are thermally connected to the components of the magnet arrangement 11 via a thermal bus comprising solid thermal conductors 39a.1 and 39a.2. It is also conceivable that the magnet arrangement 11 comprises one or more small cryogen vessels (not shown) thermally connected to the first cryocooler 31a via a similar thermal bus.

According to an embodiment, the shield tube 34 is thermally connected to the thermal radiation shield 33 via a solid thermal conductor, for example a copper braid (not shown). However, the shield tube 34 may also be thermally connected to the first cooling stage 32a.1 of the first cryocooler 31a via a solid thermal conductor.

During operation of the magnetic resonance device 10, the thermal radiation shield 33 may be maintained at an intermediate temperature, e. g. a temperature in the range of 40 K to 60 K, preferably about 43 K. The thermal radiation shield 33 may comprise an electrically conductive material configured for shielding the main magnet 12 from thermal radiation, but also from stray fields of the gradient magnetic fields generated via the gradient coils 18.

In some embodiments, the thermal radiation shield 33 and the main magnet 12 are thermally connected to the same cooling stage of the first cryocooler 31a.

In some embodiments, the thermal radiation shield 33 and the main magnet 12 are thermally and mechanically connected to both cryocoolers 31a and 31b.

In the embodiment shown in Fig. 2, the switching device comprises a heat switch 44. The heat switch 44 may be configured as a passive heat switch 44 configured to thermally connect a component of the magnet arrangement 11 to the second cryocooler 31b in dependence of a temperature dependent property of the component of the magnet arrangement 11.

In the present example, the single stage 32b.1 of the second cryocooler 31b is thermally connected to the thermal radiation shield 33 via the heat switch 44. The heat switch 44 is configured to thermally connect the single stage 32b.1 of the second cryocooler 31b to the thermal radiation shield 33 when a temperature of the thermal radiation shield 33 exceeds a predefined temperature level, and to thermally isolate the single stage 32b.1 of the second cryocooler 31b from the thermal radiation shield 33 when the temperature of the thermal radiation shield 33 is below the predefined temperature level. It is conceivable that a thermal bus element, e. g. a solid thermal conductor 39b, is arranged between the single stage 32b.1 of the second cryocooler 31b and the heat switch 44 and/or between the heat switch 44 and the thermal radiation shield 33.

The cooling stages 32a.1, 32a.2 and 32b.1 of the cryocoolers 31a and 31b may be connected to the components of the magnet arrangement 11 via solid thermal conductors 39. The magnet arrangement 11 may further comprise one or more small cryogen vessels or thermal buffers (not shown) thermally connected to the cryocooler 31a and/or the cryocooler 31b according to an embodiment described above.

In the embodiment depicted in Fig. 2, the operating temperature of the thermal radiation shield 33 thermally connected to the single stage 32b.1 of the second cryocooler 31b via the heat switch 44 exceeds a superconducting temperature of a superconducting magnet of the main magnet 12.

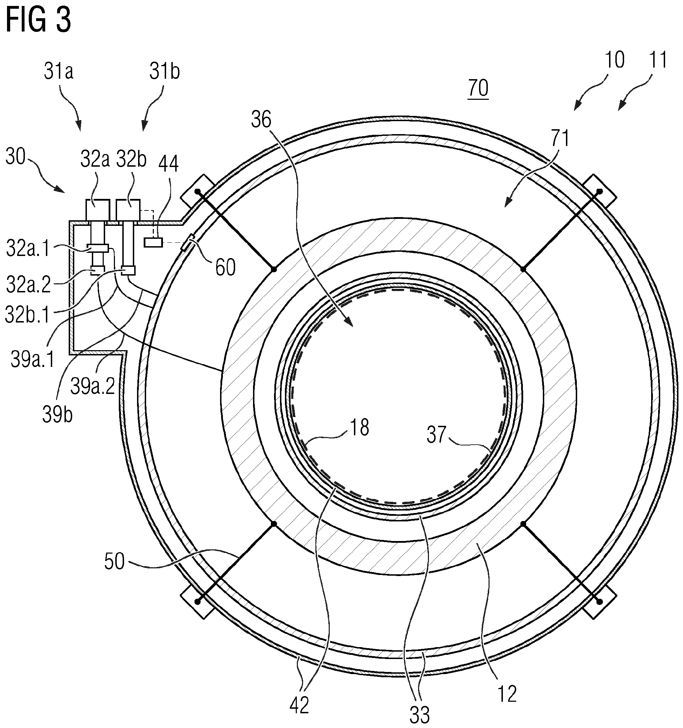

Fig. 3 shows a further embodiment of an inventive magnetic resonance device 10 comprising an inventive cryogenic system 30. In contrast to the embodiment depicted in Fig. 2, the second cryocooler 31b is mechanically and thermally connected to a component of the magnet arrangement 11. In the depicted example, the single stage 31b.1 of the second cryocooler 31b is thermally and mechanically connected to the thermal radiation shield 33 via a solid thermal conductor 39b. The switching device 38 is configured to activate the second cryocooler 31b when a temperature of the thermal radiation shield 33 exceeds a predefined temperature level, and to deactivate the second cryocooler 31b when the temperature of the thermal radiation shield 33 is below the predefined temperature level.

In the present example, the cryogenic system 30 comprises a sensor 60 configured to acquire an information indicative of a temperature dependent property of the thermal radiation shield 33 and output a signal indicative of a temperature dependent property of the thermal radiation shield 33. The signal may be transmitted from the sensor 60 to the switching device 38 via a suitable signal connection. The switching device 38 may be configured to process the signal and determine the temperature of the thermal radiation shield 33 based on the signal. It is also conceivable that the sensor 60 is configured to determine the temperature of the thermal radiation shield 33 based on the acquired information indicative of the temperature dependent property of the thermal radiation shield 33 and transmit the temperature to the switching device 38. The signals transmitted between the switching device 38 may be analog, digital, or optical signals. Is it conceivable that the switching device 38 and/or the sensor 60 comprise a control unit and/or a processing unit configured for processing any information or signal indicative of a temperature dependent property of the thermal radiation shield 33.

The switching device 38 may represent a standalone device. Alternatively, the switching device 38 may be included in the main control unit 22 of the inventive magnetic resonance device 10 (see Fig. 1). Thus, the processing unit 28 of the main control unit 22 may be used to process the signal indicative of a temperature dependent property of the thermal radiation shield 33 provided via the sensor 60.

In some embodiments, the second cryocooler 31b may be thermally and mechanically connected to further components of the magnet arrangement 11, such as the shield tube 34, a thermal buffer (not shown), but also the main magnet 12.

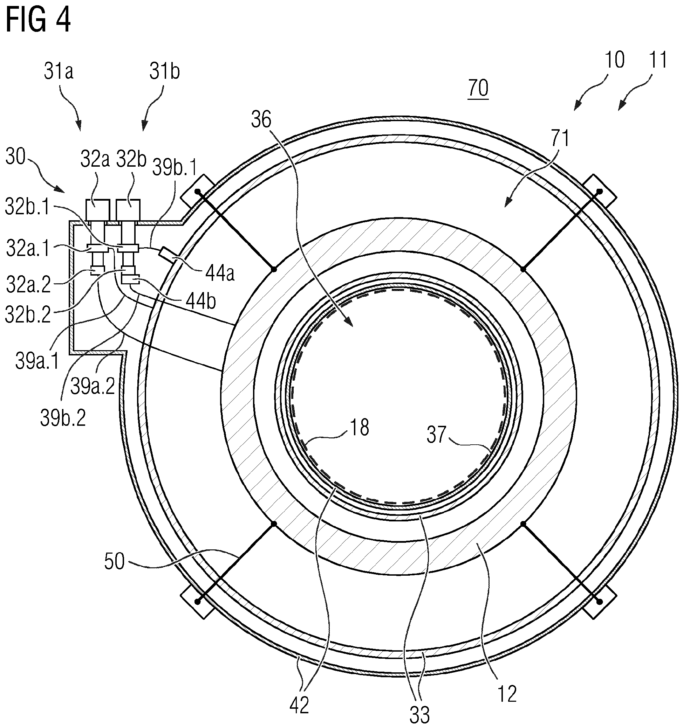

Fig. 4 depicts another embodiment of an inventive magnetic resonance device 10 comprising an inventive cryogenic system 30. In the depicted example, the second cryocooler 31b is configured as a multi-stage cryocooler comprising a first stage 32b.1 and a second stage 32b.2.

The switching device 38 comprises two heat switches 38a and 38b. The first stage 32b.1 of the second cryocooler 31b is thermally connected to the thermal radiation shield 33 via the heat switch 44a and the second stage 32b.2 of the second cryocooler 31b is thermally connected to the main magnet 12 via the heat switch 44b.

The heat switch 44a is configured to thermally connect the first stage 32b.1 of the second cryocooler 31b and the thermal radiation shield 33 when a temperature of the thermal radiation shield 33 exceeds a predefined temperature level, and to thermally isolate the first stage 32b.1 of the second cryocooler 31b from the thermal radiation shield 33 when the temperature of the thermal radiation shield 33 is below the predefined temperature level. Preferably, the heat switch 44a is configured as a passive heat switch. For example, the heat switch 44a may comprise a gas-gap heat switch or a mechanical heat switch. The predefined temperature level of the thermal radiation shield 33 may depend on or be determined by the thermal expansion of a material of the thermal radiation shield 33.

The heat switch 44b is configured to thermally connect the second stage 32b.2 of the second cryocooler 31b and the main magnet 12 when a temperature of the main magnet 12 exceeds a predefined temperature level, and to thermally isolate the second stage 32b.2 of the second cryocooler 31b from the main magnet 12 when the temperature of the main magnet 12 is below the predefined temperature level. Preferably, the heat switch 44b is configured as a passive heat switch. For example, the heat switch 44b may comprise a gas-gap heat switch or a mechanical heat switch. The predefined temperature level of the main magnet 12 may depend on or be determined by the temperature-dependent thermal expansion of a material of the thermal radiation shield 33.

In the embodiment depicted in Fig. 4, a minimum temperature level of the second stage 32b.2 of the second cryocooler 31b may be between a temperature level of the second stage 32a.2 of the first cryocooler 31a and a temperature level of the first stage 32a.1 of the first cryocooler 31a. Preferably, the temperature level of the first stage 32b.1 of the second cryocooler 31b matches or exceeds a temperature level of the first stage 32a.1 of the first cryocooler 31a.

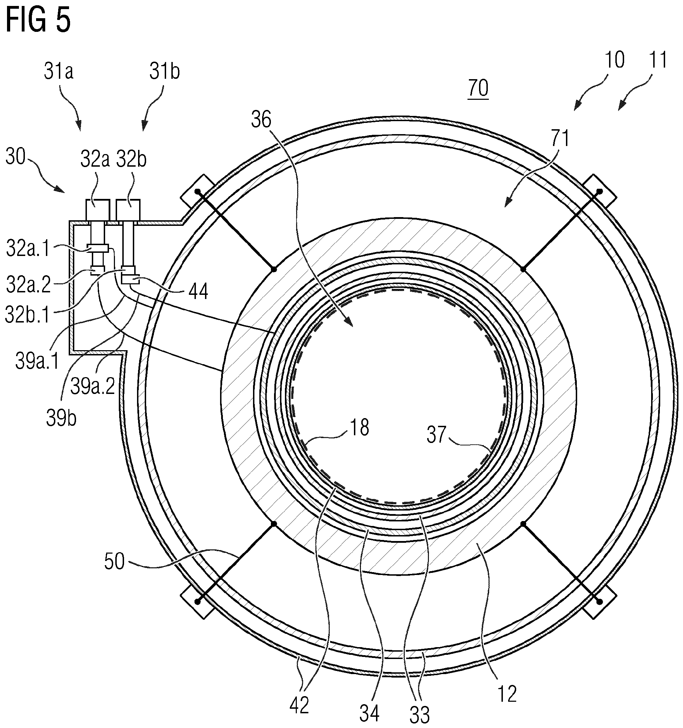

Fig. 5 depicts a further embodiment of an inventive magnetic resonance device 10 comprising an inventive cryogenic system 30. In the depicted example, the second cryocooler 31b is configured as a single-stage cryocooler comprising a single stage 32b.1. The switching device comprises a heat switch 44 arranged at the single stage 32b.1 of the second cryocooler 31b. However, it is also conceivable that the heat switch 44 is arranged at a component of the magnet arrangement 11.

The second cryocooler 31b is thermally connected to the shield tube 34 via the heat switch 44. The heat switch 44 is configured to thermally connect the single stage 32b.1 of the second cryocooler 31b to the shield tube 34 when a temperature of the shield tube 34 exceeds a predefined temperature level, and to thermally isolate the single stage 32b.1 of the second cryocooler 31b from the shield tube 34 when the temperature of the shield tube 34 is below the predefined temperature level.

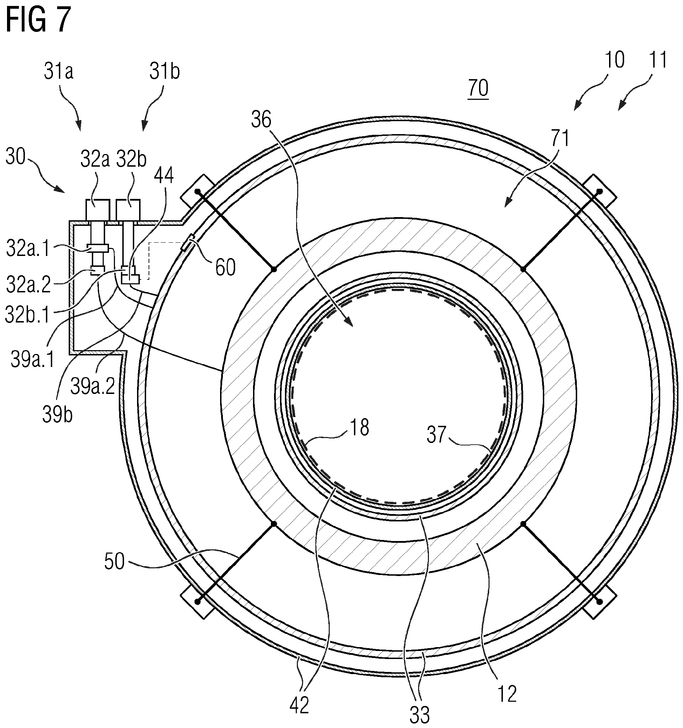

In the present example, the heat switch 44 is configured as a passive heat switch according to an embodiment described above. However, the heat switch may also be configured as an active heat switch (see Fig. 7).

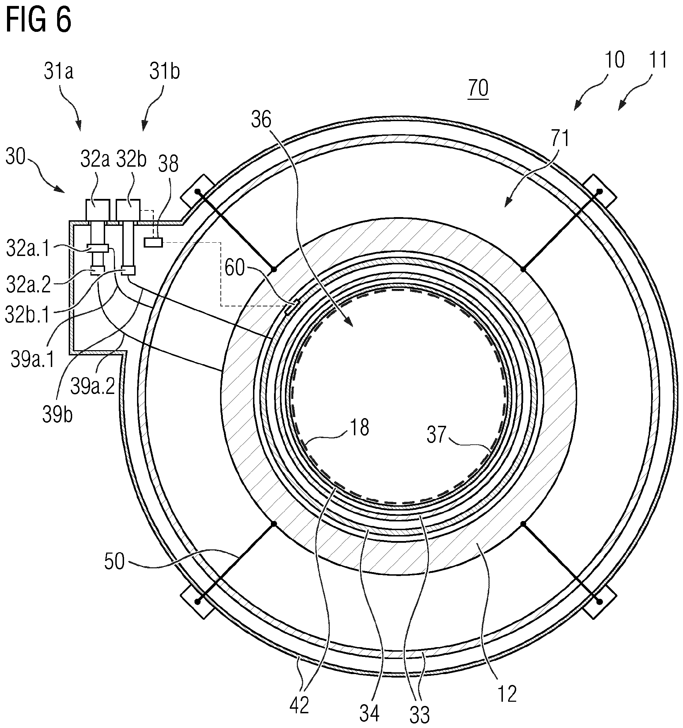

Fig. 6 shows yet another embodiment of an inventive magnetic resonance device 10 comprising an inventive cryogenic system 30. In the depicted example, the second cryocooler 31b is configured as a single-stage cryocooler comprising a single stage 32b.1. The single stage 32b.1 of the second cryocooler 31b is mechanically and thermally connected to the shield tube 34 via a solid thermal conductor 39b.

The switching device 38 is configured to activate the second cryocooler 31b when a temperature of the shield tube 34 exceeds a predefined temperature level, and to deactivate the second cryocooler 31b when the temperature of the shield tube 34 is below the predefined temperature level. The switching device 38 may comprise a sensor 60 configured to acquire an information indicative of the temperature of the shield tube 34 and a control unit configured to activate and deactivate the second cryocooler 31b in dependence of the information indicative of the temperature of the shield tube 34. In the depicted example, the sensor 60 is a temperature sensor configured to acquire the temperature of the shield tube 34 and to transmit a signal indicative of the temperature of the shield tube 34 to the switching device 38 via a signal connection (dashed line).

The switching device 38 may comprise a control unit and/or a processing unit configured to process the signal provided by the sensor 60 and to provide a control signal configured to activate or deactivate the second cryocooler 31b. In one example, the control signal provided by the switching device 38 may be configured to activate and deactivate the compressor 80b (see Fig. 1) of the second cryocooler 31b.

Of course, the second cryocooler 31b may be thermally and mechanically connected to further components of the magnet arrangement 11, such as the thermal radiation shield 33, or a thermal buffer (not shown). The cryogenic system 30 may comprise a plurality of sensors 60 configured to acquire signals indicative of the temperature of each further component of the magnet arrangement 11. The switching device 38 may be configured to activate and deactivate the second cryocooler 31b in dependence of the signals provided by the plurality of sensors 60.

According to an embodiment, the second cryocooler 31b may be configured as a multi-stage cryocooler comprising multiple cooling stages, and the switching device 38 may be configured to activate and deactivate the second cryocooler 31b in dependence of multiple predefined temperature levels, particularly multiple different predefined temperature levels, based on desired temperature levels of each component of the magnet arrangement 11 thermally and mechanically connected to the multiple cooling stages of the second cryocooler 31b.

Fig. 7 shows an embodiment of the inventive magnetic resonance 10 which is similar to the embodiment depicted in Fig. 3. In contrast to the embodiment depicted in Fig. 3, the single stage 32b.1 of the second cryocooler 31b is thermally connected to the thermal radiation shield 33 via an active heat switch 44. The active heat switch 44 is configured to thermally connect the single stage 32b.1 of the second cryocooler 31b to the thermal radiation shield 33 when a temperature of the thermal radiation shield 33 exceeds a predefined temperature level, and to thermally isolate the single stage 32b.1 of the second cryocooler 31b from the thermal radiation shield 33 when the temperature of the thermal radiation shield 33 is below the predefined temperature level.

For example, the cryogenic system 30 may comprise a sensor 60 configured to acquire an information indicative of a temperature of the thermal radiation shield 33 and output a signal indicative of the temperature of the thermal radiation shield 33. The signal may be processed by the main control unit 22 and/or the processing unit 28 of the magnetic resonance device 10 or a separate control unit of the cryogenic system 30 to determine the temperature of the thermal radiation shield 33. If the temperature of the thermal radiation shield 33 exceeds the predefined temperature level, the control unit may control an actuator element of the heat switch 44 to thermally and mechanically connect the single stage 32b.1 of the second cryocooler 31b to the shield tube 34 by moving a solid thermal conductor onto a surface of the single stage 32b.1 of the second cryocooler 31b and/or a surface the thermal radiation shield 33. In a preferred embodiment, the sensor is configured as a temperature sensor. Active heat switches similar to the one described above may also be used in the embodiments depicted in Figs. 2 and 4.

In some embodiments, a sensor 60 may be omitted and the active heat switch 44 may be controlled via the main control unit 22 of the magnetic resonance device 10 (see Fig. 1). For example, the main control unit 22 of the magnetic resonance device 10 may be configured to control the heat switch 44 to thermally connect a cooling stage of the second cryocooler 31b in dependence of an imaging sequence to be carried out via the magnetic resonance device 10. Thus, expected temperature spikes in components of the magnet arrangement 11 (e. g. due to high-performance imaging sequences) may be pre-emptively compensated by thermally connecting the cooling stage of the second cryocooler 31b to the component of the magnet arrangement 11 via the heat switch 44. The main control unit 22 may also be configured to control the heat switch 44 to thermally isolate the cooling stage of the second cryocooler 31b from the component of the magnet arrangement 11 after an imaging sequence has been carried out or when no more temperature spikes are to be expected.

The embodiments described herein are to be recognized as examples. It is to be understood that individual embodiments may be extended by or combined with features of other embodiments if not explicitly stated otherwise. The embodiments depicted in the Figures are representations that do not necessarily have to be to scale.

## Claims

1. A cryogenic system (30) for a magnetic resonance device (10), comprising a magnet arrangement (11) including at least one superconducting magnet (12), a first cryocooler (31a) thermally connected to the at least one superconducting magnet (12), a switching device (38), and a second cryocooler (31b) configured to cool a component of the magnet arrangement (11) in dependence of the switching device (38),
wherein the switching device (38) is configured to enable cooling of the component of the magnet arrangement (11) via the second cryocooler (31b) when a temperature of the component of the magnet arrangement (11) exceeds a predefined temperature level, and to disable cooling of the component of the magnet arrangement (11) via the second cryocooler (31b) when the temperature of the component of the magnet arrangement (11) is below the predefined temperature level.

2. The cryogenic system (30) according to claim 1, wherein the second cryocooler (31b) is mechanically and thermally connected to the component of the magnet arrangement (11) and wherein the switching device (38) is configured to activate the second cryocooler (31b) when the temperature of the component of the magnet arrangement (11) exceeds the predefined temperature level, and to deactivate the second cryocooler (31b) when the temperature of the component of the magnet arrangement (11) is below the predefined temperature level.

3. The cryogenic system (30) according to claim 1, wherein the switching device (38) comprises a heat switch (44), wherein the second cryocooler (31b) is thermally connected to the component of the magnet arrangement (11) via the heat switch (44) and wherein the heat switch (44) is configured to thermally connect the second cryocooler (31b) and the component of the magnet arrangement (11) when the temperature of the component of the magnet arrangement (11) exceeds the predefined temperature level, and to thermally isolate the second cryocooler (31b) from the component of the magnet arrangement (11) when the temperature of the component of the magnet arrangement (11) is below the predefined temperature level.

4. The cryogenic system (30) according to claim 3, wherein an operating temperature of the component of the magnet arrangement (11) thermally connected to the second cryocooler (31b) via the heat switch (44) exceeds a superconducting temperature of the at least one superconducting magnet (12).

5. The cryogenic system (30) according to one of the claims 3 or 4, wherein the component of the magnet arrangement (11) thermally connected to the second cryocooler (31b) via the heat switch (44) is at least one of a thermal buffer, a thermal radiation shield (33), a magnet support structure, a shield tube (34), and/or the at least one superconducting magnet (12).

6. The cryogenic system (30) according to any one of claims 3 to 5, wherein the heat switch (44) is a passive heat switch configured to thermally connect the component of the magnet arrangement (11) to the second cryocooler (31b) in dependence of a temperature dependent property of the component of the magnet arrangement (11).

7. The cryogenic system (30) according to any one of claims 3 to 5, further comprising a control unit (22) and a sensor (60), wherein the sensor (60) is configured to determine a temperature dependent property of the component of the magnet arrangement (11), wherein the heat switch (44) is an actively controlled heat switch and wherein the control unit (22) is configured to control the heat switch (44) to thermally connect the component of the magnet arrangement (11) to the second cryocooler (31b) in dependence of the temperature dependent property of the component of the magnet arrangement (11).

8. The cryogenic system (30) according to one of the preceding claims, wherein the first cryocooler (31a) is a two-stage cryocooler having a first stage and a second stage and wherein a temperature level of the first stage exceeds a temperature level of the second stage.

9. The cryogenic system (30) according to one of the preceding claims, wherein the predefined temperature level is in the range of 40 K to 120 K.

10. The cryogenic system (30) according to one of the preceding claims, wherein the second cryocooler (31b) is configured as a single-stage cryocooler comprising a single stage.

11. The cryogenic system (30) according to claim 10, wherein a temperature level of the single stage of the second cryocooler (31b) exceeds a temperature level of the second stage of the first cryocooler (31a).

12. The cryogenic system (30) according to claim 11, wherein the temperature level of the single stage of the second cryocooler (31b) matches a temperature level of the first cooling stage of the first cryocooler (31a) or exceeds the temperature level of the first stage of the first cryocooler (31a).

13. The cryogenic system (30) according to one of the claims 1 to 9, wherein the second cryocooler (31b) is a multi-stage cryocooler comprising at least a first stage and a second stage, wherein a temperature level of the first stage exceeds a temperature level of the second stage, and wherein the temperature level of the second stage of the second cryocooler (31b) exceeds a lowest temperature level provided by the first cryocooler (31a).

14. The cryogenic system (30) according to one of the claims 10 to 13, wherein a cooling capacity of a cooling stage of the second cryocooler (31b) exceeds a cooling capacity of the first stage and/or the second stage of the first cryocooler (31a).

15. A magnetic resonance device (10) configured to acquire magnetic resonance data from an object positioned in an imaging region (14) of the magnetic resonance device (10), comprising a cryogenic system (30) according to one of the preceding claims.
